# EUROPEAN PATENT SPECIFICATION

(11) **EP 2 723 267 B1**
(45) Date of publication and mention of the grant of the patent: **02.03.2016**
(21) Application number: 12730247.9
(22) Date of filing: 21.06.2012
(51) Int. Cl.: A61B 90/00, A47K 10/48, A61H 33/08, A61H 35/00, A61H 33/00, E03C 1/126, E03C 1/05, E03C 1/044

(54) **HAND CLEANING APPARATUS**
HANDREINIGUNGSVORRICHTUNG
APPAREIL DE NETTOYAGE DES MAINS

(30) Priority: 22.06.2011 GB 201110571
(43) Date of publication of application: 30.04.2014
(73) Proprietor: Wallgate Limited, Wiltshire SP2 0HB (GB)
(72) Inventor: RENFREW, Andrew Boulton, Lapley Staffordshire ST19 9JT (GB); DENBURY, Richard, Salisbury Wiltshire SP2 0HB (GB); BEALE, David, Salisbury Wiltshire SP2 0HB (GB)
(74) Representative: Perkins, Sarah
(86) International application number: PCT/GB2012/051444
(87) International publication number: WO 2012/175974

(56) References cited:
- WO-A1-87/00743
- US-A- 3 576 277
- US-A- 3 699 984
- US-A- 4 219 367
- US-A- 4 295 233

## Description

The present invention relates to hand cleaning apparatus.

A combined hand washing and drying device is described in the applicant's UK patent GB 1,365,763. The device comprises a warm water dispenser, a soap dispenser and an air dispenser intended to be used to both wash and dry the hands of a user. Devices according to GB 1,365,763 have been produced and sold and often find use in public facilities such as public lavatories and public washrooms.

In WO87/00743 a method and apparatus for dispensing flowable material is described. The functional components of the apparatus are mounted on a frame for attachment to a wall or the like and are provided under a cover which must be removed from the front of the apparatus in order to provide access to the components beneath.

Such devices are usually produced in such a way that they are mounted to a structure (e.g. an aperture in a wall of the structure or a hollow unit which is itself mounted on a wall of the structure) in order to reduce or prevent vandalism to the device. In the past, the two types of mounting have required different designs of device. Maintenance and access to the device differs according to the mounting type, and may be difficult.

There is now a need to provide hand cleaning apparatus with more convenient access for maintenance whilst maintaining a good degree of vandal-resistance.

It is an aim of the present invention to address that need.

The present invention accordingly provides hand cleaning apparatus in accordance with claim 1.

The apparatus is intended to perform one or more of the steps in hand cleaning such as hand drying or hand washing (which generally involves providing soap, providing water and subsequently performing hand drying).

The great advantage of the present invention is that it provides in a modular apparatus for hand cleaning wherein the chassis of the hand cleaning apparatus is accessible by moving the facia. Consequently, the same design of hand cleaning apparatus may enable access through the front and through the rear of the device. Thus, the apparatus according to the present invention provides more convenient access for maintenance whilst still retaining a good degree of vandal resistance. Furthermore, the modular nature of the apparatus enables parts of the apparatus to be interchanged. Thus, for example, the facia may be changed (i.e. to a facia of a different material and/or different design), or the chassis or components of the chassis may be replaced to enable speedy repair.

The frame connecting means for connecting the facia to the frame is preferably a lockable connecting means, in other words it preferably comprises a locking mechanism to lock the facia to the frame. This is advantageous because it reduces the chances of the chassis being accessed by an unauthorised person. Preferably, the locking mechanism is a spring-loaded locking mechanism.

The chassis hinge is convenient because it enables access to other parts of the chassis by, once the facia has been opened, (partially) rotating the chassis on the hinge of the chassis connecting means for access to otherwise relatively inaccessible parts of the chassis.

Generally, the chassis comprises the functional parts of the hand cleaning apparatus. The chassis comprises a hand sensor (which is preferably an infra-red hand sensor and is adapted to detect the hands of the user when within the range of the hand sensor) and an air blower (to perform at least the hand drying step in hand cleaning) and connection to a controller. Preferably, the chassis comprises the controller. For the hand cleaning apparatus according to claim 1, that is designed to both wash and dry hands of the user, the chassis further comprises a soap dispenser and a water dispenser.

The air blower will preferably be a variable speed air blower, the speed being controllable either through factory setting or by the installer/owner of the hand cleaning apparatus using the controller. Generally, the air blower will have at least three different speeds (e.g. fast, medium and slow).

The air blower may or may not provide heated air. An advantage if heated air is not provided is a reduction in power consumption by removing the need for an air heater. Instead, of providing a separate air heater the air flow from the air blower may pass through the body of the chassis thereby exchanging heat from the heat generated within the chassis (e.g. from the blower motor) and thereby warming the air above ambient. The air blower preferably comprises an impeller, in particular a relatively shallow impeller and most preferably a highspeed impeller.

If an air heater is provided it enables a relatively lower power air blower to be used.

An optional feature of the apparatus is a decontamination trap which preferably comprises a heatable vessel for collecting water draining from the apparatus and which is capable of being heated to sterilise the water in the trap.

In a preferred embodiment of the present invention, the controller is adapted to control the operation of the air blower, soap dispenser, water dispenser and/or the optional decontamination trap, preferably in response to a signal from the hand sensor.

The controller will generally comprise one or more microprocessors and memory components (e.g. non-volatile memory) and will be programmed to control the operation of the air blower, soap dispenser, water dispenser and/or decontamination trap in response to a signal from the hand sensor.

Generally, the controller will be adapted so that the operation of the air blower, soap dispenser, water dispenser and/or decontamination trap comprises the time of operation and/or the sequence of operation of one or more of these components. In other words, the controller may be adapted so that the timing of each of the component parts of the hand washing procedure and the sequence of that procedure is controllable through the controller.

A typical hand washing procedure will generally begin with insertion of the user's hands within the hand port of the apparatus. Such insertion is detected by the hand sensor which then sends a signal to the controller. This results in the operation of a typical hand-washing sequence of: soap being dispensed, a pause, water being dispensed for a set period of time, a pause and then air being blown in the hand port in order to dry the hands of the user.

If the optional decontamination trap is present, the controller may also be adapted to operate the decontamination trap at set periods of time (e.g. every 12 hours and/or particular days of the week).

Generally, the apparatus according to the invention will further comprise input means for the controller. Input means for the controller will usually comprise a control panel for providing control signals and programming e.g. the microprocessor of the controller. Such a control panel is preferably a manual control panel in communication with and/or incorporated in, the chassis. Further input means may comprise further sensors in the water, soap or air supply systems (e.g. flow rate, level and/or temperature sensors) and clock means (e.g. a real time clock and/or timing mechanism) to enable the sequence and timing of the various operations of the apparatus to be controlled.

The great benefit of manual control panels is that the owners of the apparatus are able to fully control the sequence and timing of the operation of the apparatus e.g. to reduce power or water use or, in circumstances where more extensive hand washing is necessary, to increase supply of soap, water or air during the hand washing sequence.

In a preferred embodiment, the controller is programmable to produce a hygiene flush in the apparatus at one or more predetermined time(s). The hygiene flush comprises identifying that the apparatus has not been used for a predetermined period (which may be a period selected by the installer of the apparatus and may be typically from 1 hour to 2 days or any other convenient period), and activating the water dispenser to flush the system. The hygiene flush function is particularly advantageous in situations where the apparatus has not been used for a period and it prevents or reduces water in the supply becoming stagnant and reduces growth of micro-organisms. The hygiene flush function finds particular utility in low-volume use facilities, e.g. hospitals, offices or schools (especially during holidays and the weekend). The pre-determined time is preferably set using the real time clock. Often the hygiene flush function will find use in apparatus which comprises the decontamination trap.

The hand cleaning apparatus of the invention will preferably further comprise a water heater. It is preferred if the water heater is an in-line water heater which operates (i.e. switches on) on passage of water through the heater. This is convenient because it simplifies the control of the apparatus and enables the controller to merely control the water valve without the complexity of further control of the temperature and power supply to a water heater.

The apparatus of the present invention preferably further comprises illumination means to illuminate the hands of the user in the hand port. Illumination means will usually be configured so as to illuminate the hands when they are present within the hand port and optionally to change the colour or intensity of the illumination during the sequence of operation of the apparatus. It is preferred if the sequence, timing and intensity of the illumination means is fully controllable from the controller and, in particular, from the control panel.

In order that the present invention may be better understood, it will now be described by way of example with reference to the accompanying drawings in which:
Figure 1 illustrates hand cleaning apparatus according to a first embodiment of the present invention installed in a structure; (a) illustrates the apparatus ready for use, (b) illustrates the apparatus with the facia open and (c) illustrates the apparatus with the facia open and the chassis rotated for accessibility.
Figure 2 illustrates a portion of the apparatus of Fig 1 in rear, plan view.
Figure 3 illustrates the facia of Fig 1 in (a) exploded view and (b) assembled view.
Figure 4 illustrates the frame of Fig 1 in (a) exploded and (b) assembled views.
Figure 5 illustrates the apparatus of Fig 1 in exploded view.
Figure 6 is a block diagram illustrating the control system for the apparatus.
Figure 7 illustrates hand cleaning apparatus according to a second embodiment of the present invention installed in a structure; (a) apparatus ready for use, (b) illustrates the apparatus with facia open, and (c) illustrates the apparatus with the facia open and the chassis rotated for accessibility.
Figure 8 illustrates a portion of the apparatus of Fig 7 in rear, plan view.
Figure 9 illustrates the facia of the apparatus of Fig 7 in (a) exploded view and (b) assembled view.
Figure 10 illustrates the frame of the apparatus of Fig 7 in (a) exploded and (b) assembled views.
Figure 11 illustrates the apparatus of Fig 7 in exploded view.

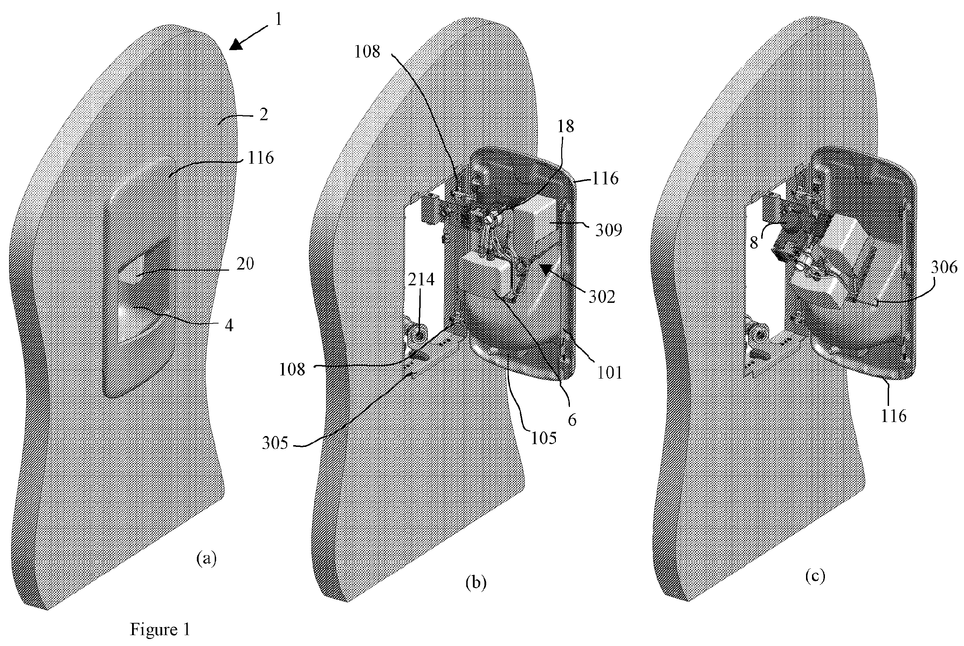

Figure 1 illustrates the hand cleaning apparatus 1 as installed in a structure 2. Structure 2 may be a wall of a building (e.g. a public convenience) or may, alternatively be a box (not shown) attached to a wall of a building. The apparatus in Figure 1a) comprises a facia 116 produced from polymeric material (for example fire resistant resin) having a hand port 4 and a shroud 20 for supply of air for drying hands, water and soap for washing.

In Figure 1(b) the hand cleaning apparatus 1 is illustrated with the facia 116 open for access to the components of the apparatus especially the main chassis 302. The facia hinges 108 are so arranged as to enable horizontal opening of the apparatus. Connected to the chassis 302 is the facia 116. The chassis 302 comprises an inline water heater 6 (which operates when water flows), soap pump assembly 18 and soap container 309. In the aperture in the frame assembly 305 is the waste connector coupler 214 which connects the drain sump 105 to the waste pipe (not shown) when the facia 116 is closed and the apparatus is ready for use. The facia hinges 108 allow easy access after the locking mechanism comprising locking strip 101 is unlocked.

Figure 1c) illustrates the chassis 302 once the chassis hinge pin 306 is released enabling the chassis 302 to rotate partly on the facia 116 thereby enabling greater access to components of the chassis 302 including soap container 309 and controller 8.

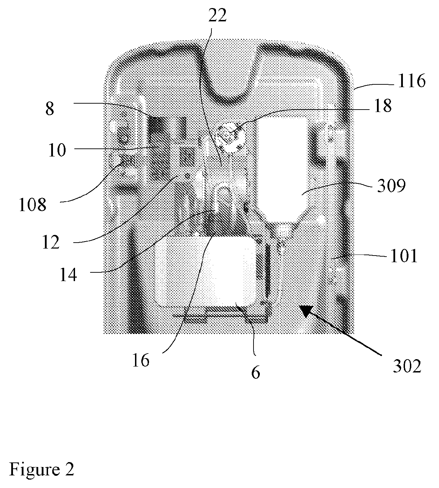

Figure 2 illustrates a rear, plan view of a cropped portion of the apparatus comprising facia 116 and chassis 302. As discussed in relation to Figure 1, the chassis 302 comprises a water heater 6, soap container 309, a soap pump assembly 18 and a controller 8. The controller 8 comprises a display panel 10 and display circuit 12 and also includes a manual control input pad for the owner/installer of the apparatus to modify the operation of the hand cleaning apparatus. The facia hinge 108 is also illustrated together with the water hose 14, water flow regulator assembly 16 and the locking strip 101.

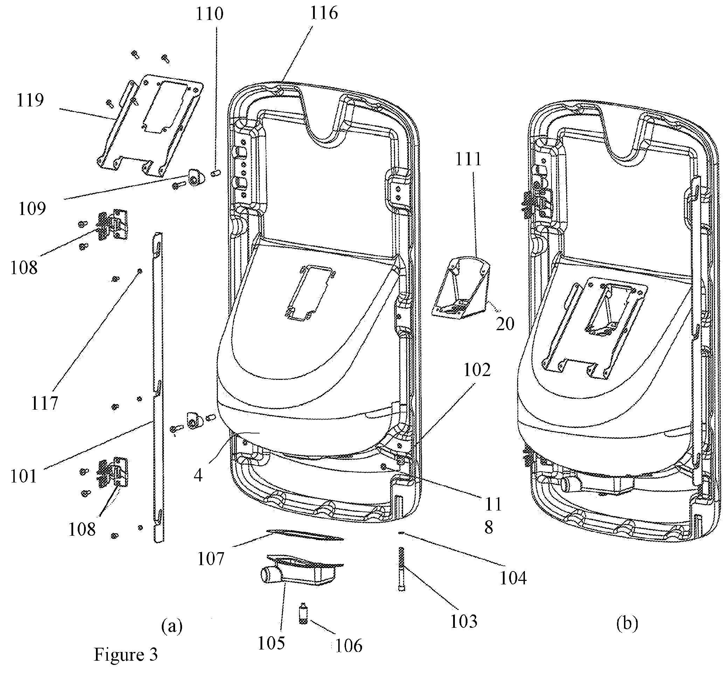

Figure 3 illustrates (a) an exploded view of the facia 116 and (b) the assembled facia. The solid surface facia 116 comprises a bowl in the hand port 4 (the rear of this feature is visible in Figure 3) for convenient hand washing by the user. Connected to the facia 116 are locking screw nut 102, circlip 104 and lock screw 103 for fixing the locking strip to the facia. The drain sump 105, drain sump fastener 106 and drain sump gasket 107 (to reduce leakage) receive waste water from the bowl of the hand port 4. The drain sump 105 may be connected to an optional decontamination unit (not shown) which is provided with a heating mechanism in order to heat and thereby sterilise waste from the hand wash procedure. The locking strip 101 is connected to the facia 116 including with lock strip spacer 117. Other components connected to the facia are chassis mount plate 119 and the hinge mechanisms including hinge side lock plate 109, lock plate sleeve 110, facia hinge 108 and various fixing mechanisms. In the front of the hand port 4 portion of the facia 116 is the nozzle housing 111 comprising the shroud 20. The nozzle housing 111 provides outlets for air, water and soap during the hand washing procedure.

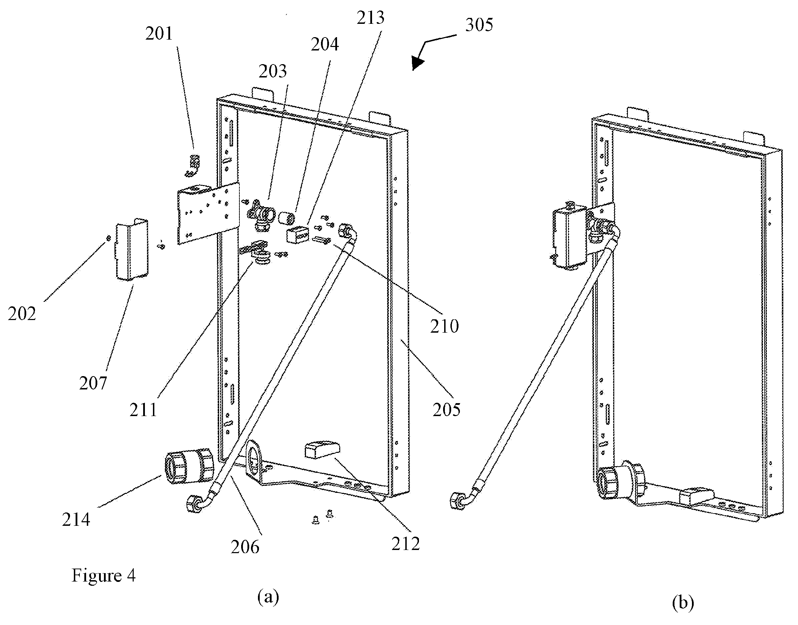

Figure 4 illustrates the frame assembly 305 in (a) exploded view and (b) assembled view. The frame assembly 305 comprises a wall frame 205 for insertion in an aperture either in a box structure attached to a surface or an aperture in e.g. the wall of a structure itself. The frame assembly 305 also comprises cable strain relief 201 and water connections comprising water inlet hose 206, water inlay elbow and back plate 203, anti-loss washer 202 and fixed elbow 204, block screw 210 and cable grip 211. Other components incorporated within the frame are waste connector coupler 214 and support block 212 and terminal block 213 and connectors to the mains power supply protected by mains input cover 207.

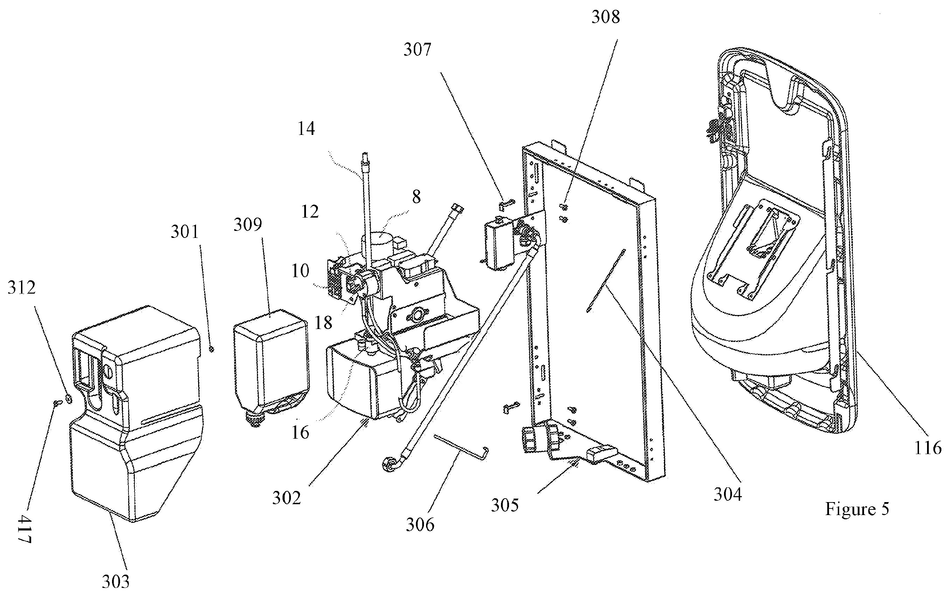

Figure 5 illustrates the apparatus in exploded form including the facia 116, the frame assembly 305, the chassis 302 and chassis cover 303. The chassis cover 303 is connected to the chassis 302 by frame screw 417 and washer 312 together with cover anti-loss washer 301. The soap container 309 is inserted into the chassis 302. The hinge release lock 307 and frame screws 308 are inserted together with the chassis stay 304 and the chassis hinge pin 306 (which as indicated in Figure 1, upon removal enables the chassis to hinge down for ready access).

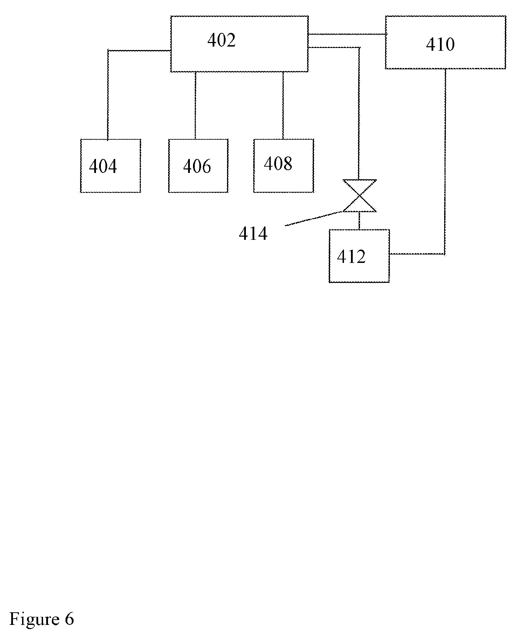

Figure 6 illustrates in schematic form the control system operated by the controller. Controller 402 comprises a microprocessor which contains non-volatile memory. The controller 402 can be modified by the display circuit and panel 410 by the user in order to adjust the sequence and timing of the various parts of the hand wash operation. The controller 402 receives input from the hand sensor circuit 404 which is connected to an infrared sensor situated in the hand port to detect the entry of hands within the hand port. The controller 402 is arranged to operate the hand dryer/air blower circuit 406 and the soap pump circuit 408 together with water valve circuit 414 according to the program set in terms of sequence and timing by the display circuit and panel 410.

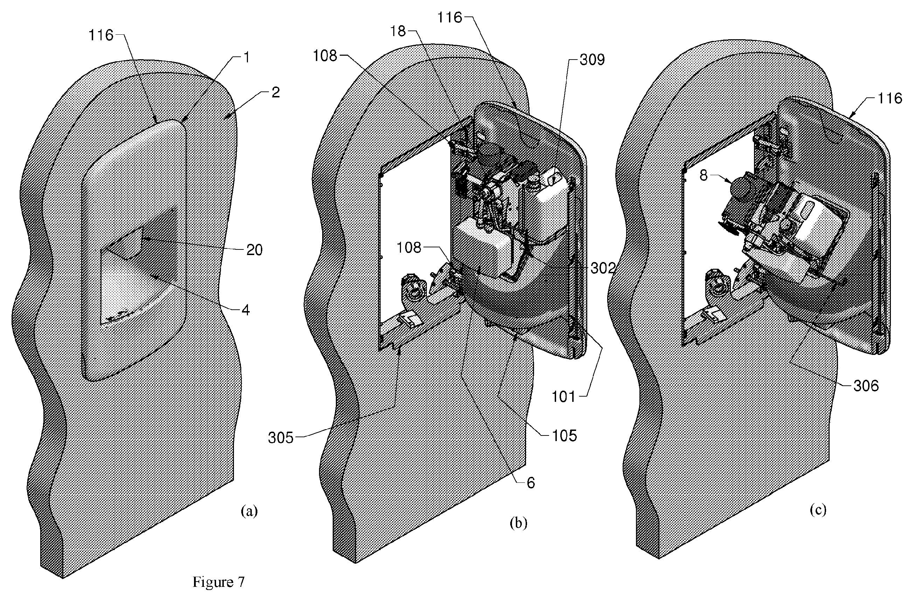

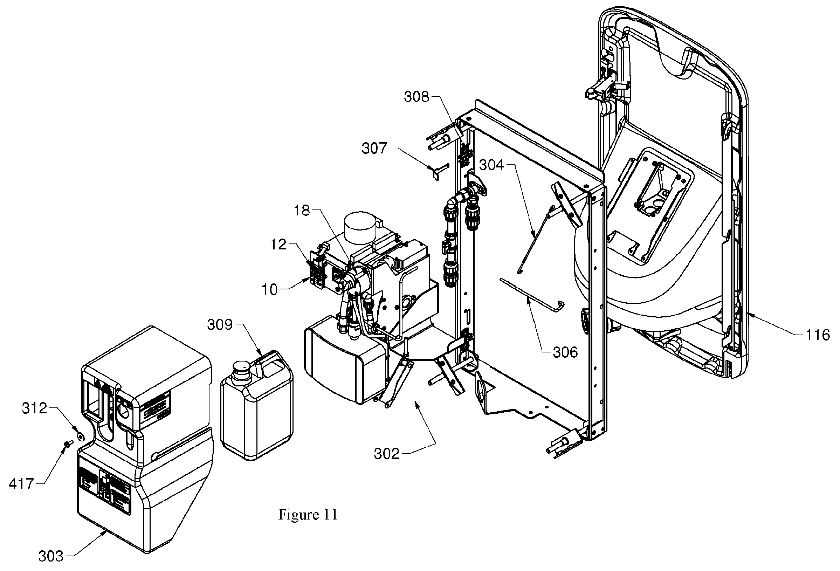

The second embodiment of the apparatus as illustrated in Figs 7 to 11 is similar to the first embodiment illustrated in Figs 1 to 5, and the description of Figs 1 to 5 generally also applies to the corresponding figures of Figs 7 to 11.

The differences are as follows. In the second embodiment, the soap container 309 is mounted so the cap is on top. This design feature simplifies manufacture, reduces leaks and improves ease of maintenance.

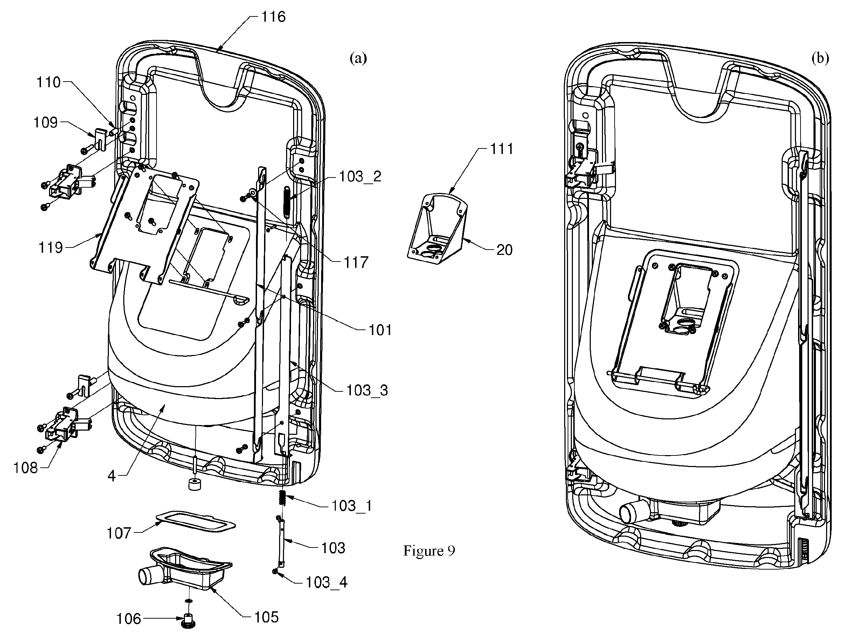

The locking strip 101 (see Fig 9) is of different design with a spring arranged so the lock activates when the facia is shut. To this end the locking mechanism comprises a lower lock spring 103_1 arranged with lock screw 103 and anti rotation pin 103_4 at the bottom of lock cam plate 103__3 with upper lock spring 103_4 positioned above. The arrangement of the locking strip 101 is such that the lock must be rotated and pushed up in order to open it, reducing the ease of opening by vandals. The anti-rotation pin 103_4 is to reduce damage to the lock when rotated. The circlip 104 is removed.

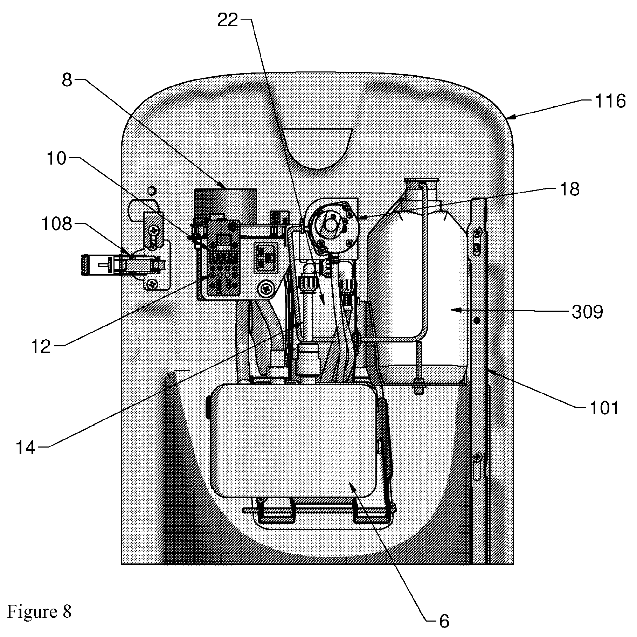

Referring to Fig. 8, the flow regulator 16 of the first embodiment has been removed in the second embodiment, because the regulator built into the water heater is often sufficient to control water flow over usual pressure ranges of the water supply. The water hose 14 is straight with an elbow to ease manufacture and maintenance.

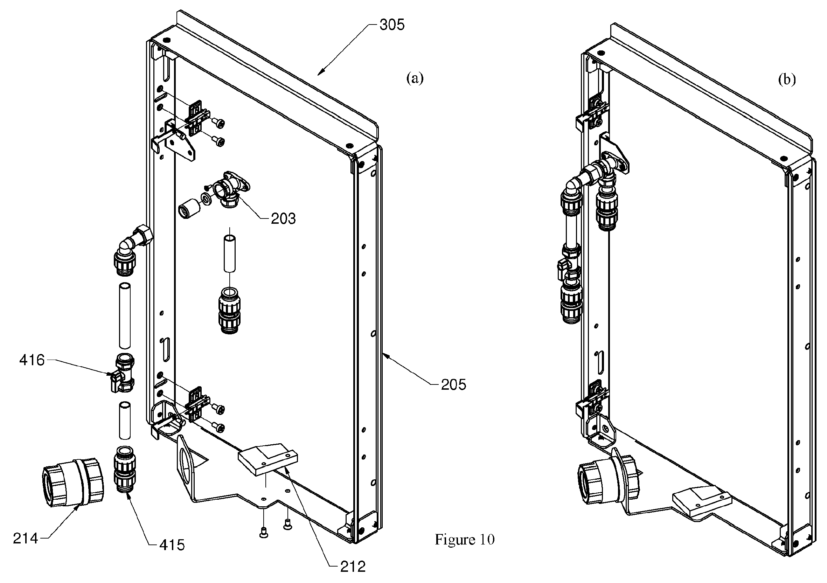

As illustrated in Fig 10, the wall frame 205 is of sheet metal (for ease of manufacture) and the support block 212 is of lower profile to improve operation. The water supply system is of different design with push fit connectors 415, a shut off valve 416 connecting to pipes, usually of copper.

In operation, a typical hand wash cycle would involve the following steps:
1) The user inserts hands into the hand port 4 which is detected by the infrared hand sensor resulting in a signal from the hand sensor circuit 404 to the controller 402.
2) The controller 402 operates the soap pump circuit 408 to release a measured quantity of soap (either foamed or unfoamed depending on how the owner/installer arranges the settings of the soap pump unit).
3) After a timed delay (typically 1 to 5 seconds) water is released via the controller operating the water valve circuit 414 for a predetermined period (typically 5 to 10 s). Flow of water through the water valve circuit 414 results in the water heater 412 (an in-line water heater with its own temperature controls) being operated and heating the water. Consequently, warm water is released into the hand port 4 from the shroud 20.
4) A time delay (typically 1 to 5 seconds) is followed by air being released by the controller 402 operating the hand dryer/air blower circuit 406. Air is provided for a predetermined time and at a predetermined flow rate as set by the owner/installer.

The air blower is preferably a high velocity air blower with or without a separate heater unit, however passage of the air through the chassis in association with the relatively warm components of the hand wash apparatus will raise the temperature of the air somewhat.

In the preferred embodiment of the invention there is a light (not shown) which acts as illumination means for hands in the hand port. The light will typically be operated upon detection of hands in the hand port through the hand sensor circuit 404 and will be operated by the controller 402. The colour and intensity of the light may vary between predetermined conditions determined by the owner/installer.

The component parts illustrated in the Figures are as follows:

### Reference Numeral

- 1: Hand cleaning apparatus
- 2: Structure
- 4: Hand port
- 6: Water heater
- 8: Controller
- 10: Display panel
- 12: Display circuit
- 14: Water hose
- 16: Water flow regulator assembly
- 18: Soap pump assembly
- 20: Shroud
- 22: Blower cover
- 101: Locking Strip
- 102: Lock Screw Nut
- 103: Lock Screw
- 103_1: Lower lock spring
- 103_2: Upper lock spring
- 103_3: Lock cam plate
- 103_4: Anti-rotation pin
- 104: Circlip
- 105: Drain Sump
- 106: Drain Sump Fastener
- 107: Drain Sump Gasket
- 108: Facia Hinge
- 109: Hinge Side Lock Plate
- 110: Lock Plate Sleeve
- 111: Nozzles Housing
- 116: Solid Surface Facia with Bowl
- 117: Lock Strip Spacer
- 118: Star Lock Washer
- 119: Chassis Mount Plate
- 201: Cable Strain Relief
- 202: Anti Loss Washer
- 203: Water Inlet Elbow and Back Plate
- 204: Fixed Elbow
- 205: Wall Frame
- 206: Water Inlet Hose
- 207: Mains Input Cover
- 210: Block Screw
- 211: Cable Grip
- 212: Support Block
- 213: Terminal Block
- 214: Waste Connector Coupler
- 301: Cover Anti Loss Washer
- 302: Main Chassis
- 303: Chassis Cover
- 304: Chassis Stay
- 305: Frame Assembly
- 306: Chassis Hinge Pin
- 307: Hinge Release Lock
- 308: Frame Screws
- 309: Soap Container
- 310: Solid Surface Facia
- 312: Washer
- 402: Controller
- 404: Hand sensor circuit
- 406: Hand dryer/air blower circuit
- 408: Soap pump circuit
- 410: Display circuit and panel
- 412: Water heater
- 414: Water valve circuit
- 415: Push fit connectors
- 416: Shut off valve
- 417: Cover screw

## Claims

1. Hand cleaning apparatus (1) comprising,
a hand sensor (404), an air blower (406) and a connection to a controller (8, 402), a soap dispenser (18, 309, 408) and a water dispenser (6, 14, 412, 414);
a frame (305);
fixing means for fixing the frame (305) to a structure;
a facia (116) comprising a hand port (4) for receiving the hands of a user; and
frame connecting means for connecting the facia (116) to the frame (305) whereby the frame connecting means comprises a facia hinge (108) connecting the facia (116) to the frame (305) whereby access to the rear of the facia (116) is provided by hinged movement around a vertical axis of the facia (116) relative to the frame (305)
and in that the hand cleaning apparatus (1) further comprises:
a chassis (302) on which is mounted said hand sensor, air blower (406) and connection to a controller (8, 402), soap dispenser (18, 309, 408) and water dispenser (6, 14, 412, 414); and
chassis connecting means for connecting the chassis to the facia (116), the chassis connecting means comprising a chassis hinge connecting the chassis (302) to the rear of the facia (116) whereby greater access to said hand sensor, air blower and connection to a controller (8, 402), soap dispenser and water dispenser is provided by rotational movement around a horizontal axis of the chassis (302) relative to the rear of the facia (116).

2. Hand cleaning apparatus as claimed in claim 1, wherein the frame connecting means comprises a locking mechanism (101, 102, 103, 104) to lock the facia (116) to the frame (305).

3. Hand cleaning apparatus as claimed in claim 1 wherein the chassis connecting means comprises a locking mechanism (304, 307).

4. Hand cleaning apparatus as claimed in any one of the preceding claims, further comprising a decontamination trap.

5. Hand cleaning apparatus as claimed in claim 1, wherein the air blower (406) is a variable speed air blower.

6. Hand cleaning apparatus as claimed in claim 1, wherein the controller (8, 402) is adapted to control the operation of the air blower (406) and/or a soap dispenser (408) and/or a water dispenser (414) and/or a decontamination trap in response to a signal from the hand-sensor (404).

7. Hand cleaning apparatus as claimed in claim 6, wherein operation of the air blower (406), soap dispenser (408), water dispenser (414) and/or decontamination trap comprises the time of operation and/or sequence of operation thereof.

8. Hand cleaning apparatus as claimed in either claim 6 or claim 7, further comprising input means for the controller (8, 402).

9. Hand cleaning apparatus as claimed in any one of claims 6 to 8, wherein the controller (8, 402) is programmable to produce a hygiene flush at a predetermined time.

10. Hand cleaning apparatus as claimed in any one of claims 1 to 9, further comprising a water heater (6).

11. Hand cleaning apparatus as claimed in any one of the preceding claims, further comprising illumination means to illuminate the hands of a user in the hand port (4).

## Patentansprüche

1. Handreinigungsgerät (1), umfassend:
einen Handsensor (404), ein Luftgebläse (406) und ein Anschluss zu einer Steuerungseinrichtung (8, 402), eine Ausgabeeinrichtung für Seife (18, 309, 408) und eine Ausgabeeinrichtung für Wasser (6, 14, 412, 414);
einen Rahmen (305);
eine Befestigungsvorrichtung zum Befestigen des Rahmens (305) an einem Baukörper;
eine Blende (116) mit einer Handöffnung (4) zum Aufnehmen der Hände eines Benutzers; und
eine Rahmenverbindungsvorrichtung zum Verbinden der Blende (116) mit dem Rahmen (305),
wobei die Rahmenverbindungsvorrichtung ein Blendenscharnier (108) umfasst, das die Blende (116) mit dem Rahmen (305) verbindet, wobei der Zugang zum Bereich hinter der Blende (116) durch die Scharnierbewegung der Blende (116) um eine vertikale Achse in Bezug auf den Rahmen (305) ermöglicht wird, und wobei das
Handreinigungsgerät (1) des Weiteren umfasst:
ein Gestell (302), an dem der Handsensor, das Luftgebläse (406) und der Anschluss zu einer Steuerungseinrichtung (8, 402), die Ausgabeeinrichtung für Seife (18, 309, 408) und die Ausgabeeinrichtung für Wasser (6, 14, 412, 414) befestigt sind; und eine Gestellverbindungseinrichtung zum Verbinden des Gestells mit der Blende (116), die Gestellverbindungseinrichtung umfassend ein Gestellscharnier zum Verbinden des Gestells (302) mit der Rückseite der Blende (116), wodurch ein erweiterter Zugang zu dem Handsensor, dem Luftgebläse und dem Anschluss zu einer Steuerungseinrichtung (8, 402), der Ausgabeeinrichtung für Seife und der Ausgabeeinrichtung für Wasser durch die Rotationsbewegung des Gestells (302) um eine horizontale Achse in Bezug auf die Rückseite der Blende (116) ermöglicht wird.

2. Handreinigungsgerät gemäß Anspruch 1, wobei die
Rahmenverbindungsvorrichtung einen Verriegelungsmechanismus (101, 102, 103, 104) zum Verriegeln der Blende (116) am Rahmen (305) umfasst.

3. Handreinigungsgerät gemäß Anspruch 1, wobei die Gestellverbindungseinrichtung einen Verriegelungsmechanismus (304, 307) umfasst.

4. Handreinigungsgerät gemäß einem der vorherigen Ansprüche, des Weiteren umfassend einen Dekontaminierungssiphon.

5. Handreinigungsgerät gemäß Anspruch 1, wobei es sich bei dem Luftgebläse (406) um ein Luftgebläse mit variabler Drehzahl handelt.

6. Handreinigungsgerät gemäß Anspruch 1, wobei die Steuerungseinrichtung (8, 402) dazu eingerichtet ist, die Betätigung des Luftgebläses (406) und/oder einer Ausgabeeinrichtung für Seife (408) und/oder einer Ausgabeeinrichtung für Wasser (414) und/oder eines Dekontaminierungssiphons als Reaktion auf ein Signal vom Handsensor (404) zu steuern.

7. Handreinigungsgerät gemäß Anspruch 6, wobei die Betätigung des Luftgebläses (406), der Ausgabeeinrichtung für Seife (408), der Ausgabeeinrichtung für Wasser (414) und/oder des Dekontaminierungssiphons den Betätigungszeitpunkt und/oder die Reihenfolge ihrer Betätigung umfasst.

8. Handreinigungsgerät gemäß einem der Ansprüche 6 oder 7, des Weiteren umfassend eine Eingabeeinrichtung für die Steuerungseinrichtung (8, 402).

9. Handreinigungsgerät gemäß einem der Ansprüche 6 bis 8, wobei die Steuerungseinrichtung (8, 402) programmierbar ist, zu einem vorgegebenen Zeitpunkt eine Hygienespülung durchzuführen.

10. Handreinigungsgerät gemäß einem der Ansprüche 1 bis 9, des Weiteren umfassend eine Wasserheizung (6).

11. Handreinigungsgerät gemäß einem der vorherigen Ansprüche, des Weiteren umfassend eine Beleuchtungseinrichtung zum Beleuchten der Hände eines Benutzers in der Handöffnung (4).

## Revendications

1. Un appareil pour laver les mains (1) comprenant :
un détecteur de main (404), une souffleuse d'air (406) et un raccord à un dispositif de commande (8, 402), un distributeur de savon (18, 309, 408) et un distributeur d'eau (6, 14, 412, 414) ;
un support (305) ;
un moyen de fixation pour fixer le support (305) sur une structure ;
une façade (116) comprenant un orifice pour les mains (4) pour recevoir les mains d'un utilisateur ; et
un moyen de raccord de support pour raccorder la façade (116) au support (305) grâce à quoi le moyen de raccord de support comprend une articulation de façade (108) raccordant la façade (116) au support (305) grâce à quoi un accès à l'arrière de la façade (116) est fourni par un déplacement articulé sur un axe vertical de la façade (116) relativement au support (305)
et en ce que l'appareil pour laver les mains (1) comprend en outre :
un châssis (302) sur lequel sont montés lesdits détecteur de main, souffleuse d'air (406) et raccord à un dispositif de commande (8, 402), distributeur de savon (18, 309, 408) et distributeur d'eau (6, 14, 412, 414) ; et
un moyen de raccord de châssis pour raccorder le châssis à la façade (116), le moyen de raccord de châssis comprenant une articulation de châssis raccordant le châssis (302) à l'arrière de la façade (116) grâce à quoi un accès plus important auxdits détecteur de main, souffleuse d'air et raccord à un dispositif de commande (8, 402), distributeur de savon et distributeur d'eau est fourni par déplacement rotatif sur un axe horizontal du châssis (302) relativement à l'arrière de la façade (116).

2. Appareil pour laver les mains tel que revendiqué dans la revendication 1, dans lequel le moyen de raccord de support comprend un mécanisme de verrouillage (101, 102, 103, 104) pour verrouiller la façade (116) sur le support (305).

3. Appareil pour laver les mains tel que revendiqué dans la revendication 1 dans lequel le moyen de raccord de châssis comprend un mécanisme de verrouillage (304, 307).

4. Appareil pour laver les mains tel que revendiqué dans l'une quelconque des revendications précédentes, comprenant en outre un siphon de décontamination.

5. Appareil pour laver les mains tel que revendiqué dans la revendication 1, dans lequel la souffleuse d'air (406) est une souffleuse d'air à vitesse variable.

6. Appareil pour laver les mains tel que revendiqué dans la revendication 1, dans lequel le dispositif de commande (8, 402) est conçu pour commander le fonctionnement de la souffleuse d'air (406) et/ou d'un distributeur de savon (408) et/ou d'un distributeur d'eau (414) et/ou d'un siphon de décontamination en réponse à un signal du détecteur de main (404).

7. Appareil pour laver les mains tel que revendiqué dans la revendication 6, dans lequel le fonctionnement de la souffleuse d'air (406), du distributeur de savon (408), du distributeur d'eau (414) et/ou du siphon de décontamination comprend la durée de fonctionnement et/ou une séquence de fonctionnement de ceux-ci.

8. Appareil pour laver les mains tel que revendiqué dans la revendication 6 ou la revendication 7, comprenant en outre un moyen d'entrée pour le dispositif de commande (8, 402).

9. Appareil pour laver les mains tel que revendiqué dans l'une quelconque des revendications 6 à 8, dans lequel le dispositif de commande (8, 402) peut être programmé pour produire un rinçage hygiénique à un moment prédéterminé.

10. Appareil pour laver les mains tel que revendiqué dans l'une quelconque des revendications 1 à 9, comprenant en outre un chauffe-eau (6).

11. Appareil pour laver les mains tel que revendiqué dans l'une quelconque des revendications précédentes, comprenant en outre un moyen d'éclairage pour éclairer les mains d'un utilisateur dans l'orifice pour les mains (4).
